# EUROPEAN PATENT SPECIFICATION

(11) **EP 4 466 063 B1**
(45) Date of publication and mention of the grant of the patent: **18.11.2026**
(21) Application number: 23743910.4
(22) Date of filing: 19.01.2023
(51) Int. Cl.: A61N 1/36, A61N 1/04, A61F 5/01

(54) **SYSTEMS FOR INTEGRATING ELECTRONICS AND SENSORS INTO A WEARABLE THERAPEUTIC JOINT SUPPORT GARMENT**
SYSTEME ZUR INTEGRATION VON ELEKTRONIK UND SENSOREN IN EIN TRAGBARES THERAPEUTISCHES GELENKUNTERSTÜTZUNGSKLEIDUNGSSTÜCK
SYSTÈMES D'INTÉGRATION DE COMPOSANTS ÉLECTRONIQUES ET DE CAPTEURS DANS UN VÊTEMENT DE SUPPORT D'ARTICULATION THÉRAPEUTIQUE À PORTER SUR SOI

(30) Priority: 21.01.2022 US 202263301664 P
(43) Date of publication of application: 27.11.2024
(73) Proprietor: Motive Health, Inc., Scottsdale, AZ 85260 (US)
(72) Inventor: PAULKE, Gabor, Scottsdale, Arizona 85255 (US); FIG, Ryan, Scottsdale, Arizona 85255 (US); HART, Erin, Scottsdale, Arizona 85255 (US); SHAHRIARI, Kereshmeh, Scottsdale, Arizona 85255 (US); JHALA, Dharmavirsinh, Scottsdale, Arizona 85255 (US); WU, Daniel, Scottsdale, Arizona 85255 (US); COLEMAN, Struan, Scottsdale, Arizona 85255 (US)
(74) Representative: WBH Wachenhausen Patentanwälte PartG mbB
(86) International application number: PCT/US2023/060914
(87) International publication number: WO 2023/141512

(56) References cited:
- WO-A2-2018/098046
- US-A1- 2004 044 384
- US-A1- 2004 044 384
- US-A1- 2007 106 343
- US-A1- 2007 106 343
- US-A1- 2018 318 583
- US-A1- 2018 318 583
- US-A1- 2021 260 371
- US-A1- 2021 260 371
- US-B2- 10 179 238
- US-B2- 7 899 556
- US-B2- 7 899 556

## Description

### BACKGROUND

Joint support wraps are used for improving stability in body joints such as knees and shoulders. However, conventional body joint wrap designs are cumbersome and typically do not include therapeutic electronics. Joint support wraps with embedded electronics do not effectively address comfort, bilateral use (e.g. right and left joints), washability, and ease of manufacturing considerations. Rigid bodies embedded in a wrap may not allow for a form fit around a joint, may be costly to manufacture, and are limited to allow for integration of multiple electronics boards, sensors, and wires. In addition, rigid electronic structures may cause undue pressure on the joints which can lead to additional discomfort and damage instead of a healing effect.

Therefore, there is a need in the art for a system that integrates electronics for wearable garments including a joint wrap, a band, and/or other wearable support device into a flexible form to enclose electronics needed for delivery of neuromodulation modalities including Neuromuscular Electrical Stimulation (NMES), Transcutaneous Electrical Nerve Stimulation (TENS), combined NMES and TENS waveform, thermotherapy or heat therapy, and/or sensors needed to detect bio-signals and other health data.
US 2018/0318583 A1 relates to systems and apparatus for gait modulation and methods of use. US 2004/0044384 A1 relates to a therapeutic method and apparatus.
US 7,899,556b2 relates to an orthosis for a gait modulation system.
US 2021/0260371 A1 relates to patient therapy systems and methods.
US 2007/0106343 A1 relates to an apparatus for applying electrical current to the neuromuscular system.
WO 2018/098046 A2 relates to haptic human machine interface and wearable electronics methods and apparatus.
US 10,179,238 B2 relates to systems for peripheral nerve stimulation.

### SUMMARY

The invention is set out in the appended set of claims.

In some embodiments, the disclosure is directed to a system for integrating a removable electrical panel into a garment for the delivery of neuromuscular stimulation. In some embodiments, the system comprises one or more of a garment, one or more electrodes, and at least one controller. In some embodiments, the garment is configured to wrap around a biological joint. Non-limiting examples of biological joints include a human knee, elbow, ankle, wrist, and the like. In some embodiments, the one or more electrodes are coupled to the garment. In some embodiments, the at least one controller is coupled to the one or more electrodes. In some embodiments, the at least one controller is configured to send one or more electrical pulses to the one or more electrodes while the garment is wrapped around the biological joint. In some embodiments, the one or more electrical pulses are configured to stimulate one or more of a nerve and a muscle in an area of the biological joint.

In some embodiments, the system further comprises an electrical panel. In some embodiments, the at least one controller is configured to transmit electrical power to one or more electrical contacts. In some embodiments, the electrical contacts are integrated into the electrical panel. In some embodiments, the one or more contacts are configured to couple to one or more electrodes. In some embodiments, the one or more contacts are configured to couple to one or more electrical heating and/or cooling elements. In some embodiments, the one or more electrodes are coupled to the electrical panel. In some embodiments, the electrical panel is coupled to the garment. In some embodiments, the electrical panel is configured to securely couple to the garment. In some embodiments, the electrical panel is configured to be removably coupled to the garment. In some embodiments, the electrical panel comprises an electrical circuit. In some embodiments, the electrical circuit is configured to transmit the one or more electrical pulses to the one or more electrodes. In some embodiments, the electrical panel is a flexible electrical panel. In some embodiments, the flexible electrical panel is configured to at least partially conform to a shape of the garment when the garment is deformed during a wrapping around the biological joint.

In some embodiments, the electrical panel is configured to be detachably removable from the garment. In some embodiments, at least a portion of the electrical panel is configured to pass through a hollow portion of the garment. In some embodiments, the electrical panel comprises at least one controller coupling. In some embodiments, the at least one controller coupling is configured to surround at least a portion of the at least one controller. In some embodiments, the at least one controller is configured to be detachably coupled to the at least one controller coupling.

In some embodiments, the garment comprises a main body and one or more arms. As used herein the terms "arm" and "finger" are not a reference to a human feature but are instead defined as an elongated material extending away from a central location that has a shape that distinguishes the elongated material from other material features at the main body and/or central portion of the overall structure. In some embodiments, the one or more arms extend away from the main body. In some embodiments, the main body is configured and arranged to extend past a parameter of the electrical panel. In some embodiments, the main body comprises a hollow portion. In some embodiments, one or more arms comprise a hollow portion. In some embodiments, at least one of the at least one controller and the at least one controller coupling are configured to pass through a hollow portion when the electrical panel is coupled to the garment and/or main body.

In some embodiments, the one or more arms are two arms. In some embodiments, a first arm of the two arms comprises a first length. In some embodiments, a second arm of the two arms comprises a second length. In some embodiments, the first length is different from the second length.

In some embodiments, the electrical panel comprises at least one controller coupling. In some embodiments, the at least one controller coupling is configured to surround at least a portion of the at least one controller. In some embodiments, the at least one controller is configured to be detachably coupled to the at least one controller coupling. In some embodiments, at least one of the at least one controller and the at least one controller coupling are configured to pass through the hollow portion of the garment (e.g., main body) when the electrical panel is coupled to the garment (e.g., main body).

In some embodiments, the electrical circuit is a flexible electrical circuit. In some embodiments, the flexible electrical circuit includes one or more electrode couplings. In some embodiments, the one or more electrode couplings are each configured to enable removable electrical coupling of each of the one or more electrodes.

In some embodiments, each of the one or more electrode couplings comprise one or more flexible fingers extending from a center of the one or more electrode couplings. In some embodiments, the one or more flexible fingers are configured to prevent a bunching of the electrical panel in an area of the one or more flexible fingers when the flexible electrical panel is deformed with a deformation of the garment. In some embodiments, the one or more flexible fingers are configured to (at least partially) prevent a creasing of the electrical panel in the area of a contact. In some embodiments, the one or more fingers are configured to at least partially elastically aid in returning the panel to a flat (or flatter shape) when the garment is deformed into a flat shape.

In some embodiments, the flexible electrical circuit comprises one or more electricity flow paths. In some embodiments, the one or more electricity flow paths are configured to deliver electricity from the at least one controller to the one or more electrode couplings. In some embodiments, at the one or more electricity flow paths are configured to extend along a finger length of at least one of the one or more flexible fingers at each of the one or more electrode couplings. In some embodiments, the flexible electrical circuit, the one or more electricity flow paths, and/or the one or more flexible fingers are housed within an internal portion of the electrical panel. In some embodiments, the internal portion includes a waterproof material, such as thermally formed foam, to protect the flexible electrical circuit.

### DRAWINGS DESCRIPTION

FIG. 1 shows a wearable garment that includes a unique wrap or band for placement on or around a user's joint according to some embodiments.
FIG. 2 illustrates an electrical panel overview according to some embodiments.
FIG. 3 shows an electrode wiring diagram according to some embodiments.
FIG. 4 depicts rendered views of the assembled electrical panel as well as the thermoformed waterproof electrical panel according to some embodiments.
FIG. 5 shows options for connecting the dock board to the flexible circuit as well as a rendering of the flexible circuit material.
FIGs. 6 depicts various components of the garment as a joint wrap according to some embodiments.
FIG. 7 shows a an assembled garment joint wrap according to some embodiments.
FIG. 8 illustrates how the joint wrap is configured to secure around a user's joint according to some embodiments.
FIG. 9 illustrates a computer system enabling or comprising the systems and methods in accordance with some embodiments of the system.
FIG. 10 depicts an NMES Pulse Signal profiles executed by the system and implemented by one or more electrodes according to some embodiments.
FIG. 11 shows a TENS pulse signal profile executed by the system and implemented by one or more electrodes according to some embodiments.
FIG. 12 shows a front view of the wrap system according to some embodiments.
FIG. 13 shows a rear view of the wrap system according to some embodiments.
FIG. 14 is a closeup perspective view of FIG. 13 according to some embodiments.
FIG. 15 is perspective view of FIG. 12 according to some embodiments.
FIG. 16 illustrates a wrapped view of the system according to some embodiments.
FIG. 17 is a line drawing with various labels describing aspects of FIGs. 12 and 13 according to some embodiments.
FIG. 18 shows various non-limiting example dimensions of FIG. 12 according to some embodiments.
FIG. 19 depicts various non-limiting example dimensions of the wrap system of FIG. 13 according to some embodiments.
FIG. 20 shows various aspects of a front portion of the electrical panel according to some embodiments.
FIG. 21 shows various aspects of a rear portion of the electrical panel according to some embodiments.
FIG. 22 shows various outlines and dimensions of the front and rear portions of the electrical panel according to some embodiments.

### DETAILED DESCRIPTION

In some embodiments, the disclosure is directed to system that includes a garment that is configured to deliver electrical impulses to one or more portions of a user's skin. In some embodiments, the garment comprises a removable electrical panel. In some embodiments, the garment and/or electrical panel comprises a removable controller. In some embodiments, the removable controller comprises one or more computers comprising one or more processors and one or more non-transitory computer readable media. In some embodiments, the one or more non-transitory computer readable media comprise program instructions stored thereon that when executed cause the one or more computers to implement one or more steps. In some embodiments, a program step includes instructions to establish, by the one or more processors, a connection to one or more other computers. In some embodiments, a program step includes instruction to receive, by the one or more processors, one or more electrical impulse and/or electrical power transmission instructions. In some embodiments, a program step includes instructions to execute, by the one or more processors, a therapeutic electrical profile. In some embodiments, the therapeutic electrical profile is configured to deliver electrical impulses to one or more electrodes coupled to the garment and/or electrical panel.

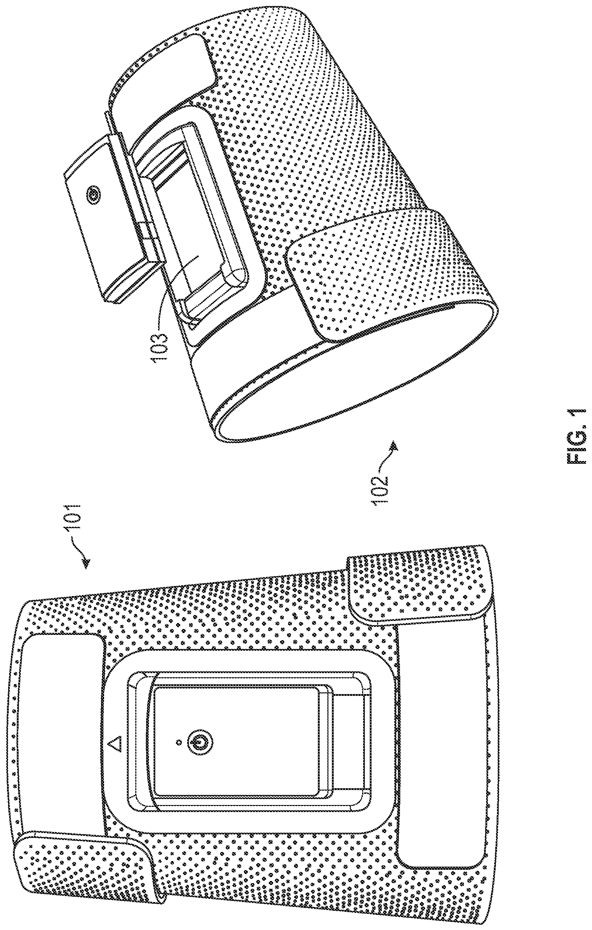

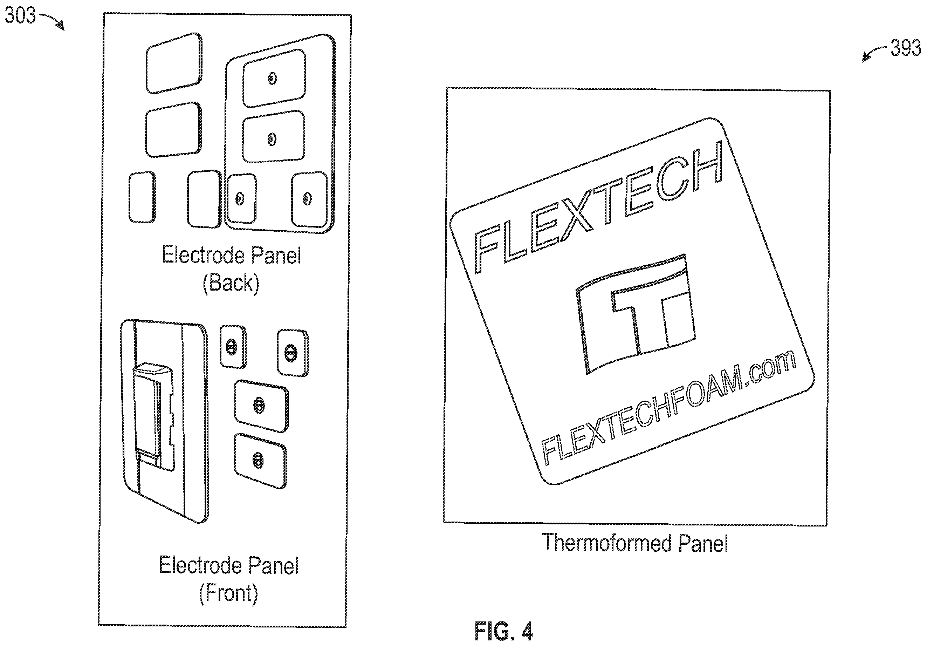

FIGs. 1 and 4 show a wearable garment 101 that include a unique wrap or band 102 with a removable electrical panel (FIG 4) for placement on a user's joint (e.g., joint) according to some embodiments of the system. In some embodiments, the system includes one or more electrical panels 103 that each include snap-type electrodes configured for placement over one or more portions of a joint (e.g. joint). In some embodiments, the electrical panel 103 includes a unique electrode mounting area that includes electrodes for NMES or TENS and/or any other neuromodulation stimulation of the muscles or soft tissue (e.g., surrounding a joint).

In some embodiments, the electrical panel 103 includes conductive textile electrodes that are integrated in or detachable from the electrical panel. In some embodiments, the system includes a removable electrical panel 103 that includes one or more electronics, electrodes, and/or sensors. In some embodiments, the system comprises one or more electrical panels, where each panel position is adjustable to one or more panel locations on the main body *. In some embodiments, the electrical panel 103 includes a foam substrate (e.g., Lycra^{®}/foam) that includes electronics and/or printed flexible wires, examples of which are further described herein. In some embodiments, the system includes a wired or wireless snap-on controller that includes a pulse generator configured to implement electrical stimulation therapies and thermotherapy and/or heat therapy. In some embodiments, the system includes one or more sensors including one or more of an embedded force gauge, accelerometer, electromyogram (EMG) sensor, and pressure gauge in the electrical panel to enable the controller to detect one or more of motion, the activation forces of muscles surrounding a joint, bio-signals (EMG), bioimpedance, and/or skin temperature. In some embodiments, the system includes a graphical user interface (GUI) configured to display activation levels of muscles, detected MEG signal, motion, bioimpedance, and body temperature for the user. In some embodiments, the system is configured to calculate the minimum required NMES stimulation intensities for muscles based on the activation force.

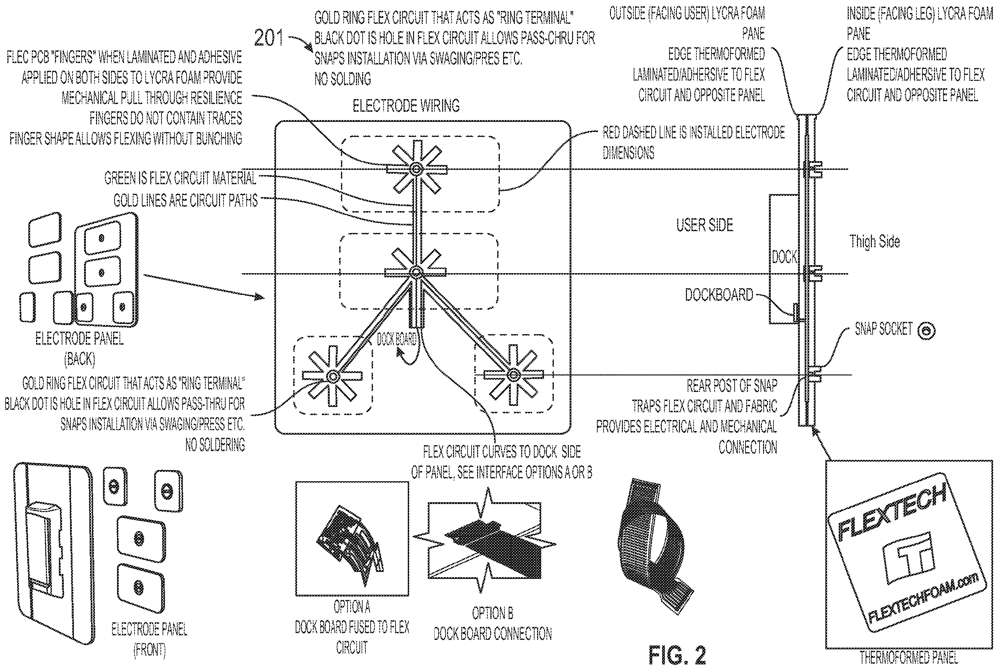

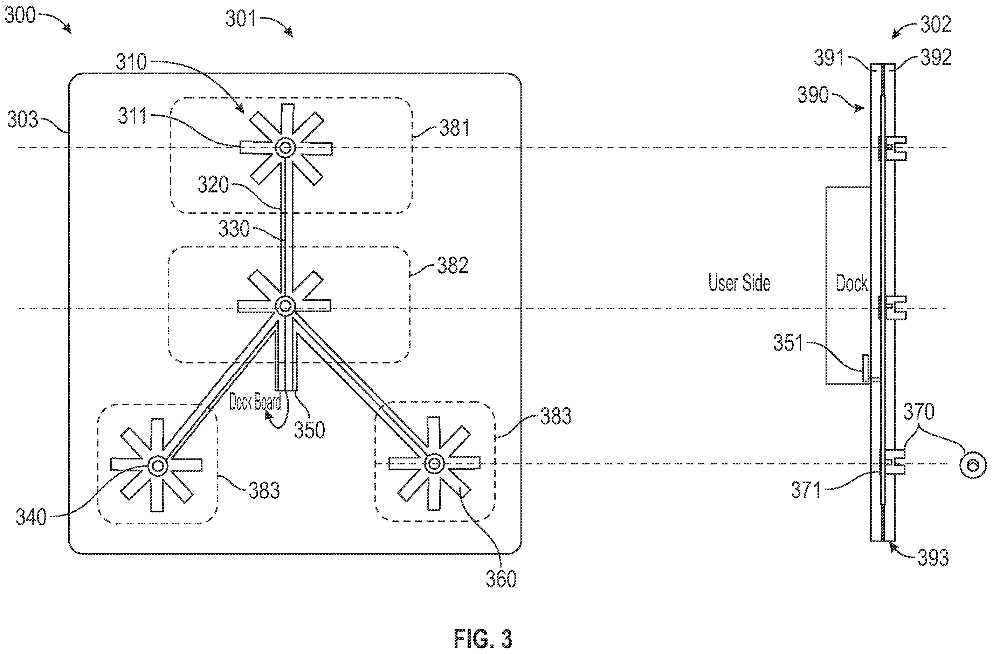

FIG. 2 illustrates a system overview 201 according to some embodiments. FIG. 3 shows an electrode wiring diagram 300 according to some embodiments. In some embodiments, front view 301 and side view 302 depict a non-limiting configuration for electrodes and/or electrical circuits on and/or within the electrical panel 303. In some embodiments, the electrical panel 303 comprises a flexible circuit 310 comprising one or more flexible printed circuit board (PCB) fingers 311. In some embodiments, the flexible circuit 310 comprises laminate and/or adhesive applied to one or both sides and/or the foam 390. In some embodiments, the laminate and/or adhesive is configured to hold in place one or more of the PCD, the one or more contacts, and two opposing faces of the electrical panel. In some embodiments, the foam 390 comprises Lycra^{®} foam. In some embodiments, the flexible circuit 310 is positioned between a user side foam panel 391 and a body side foam panel 392. In some embodiments, one or both panels 391, 392 comprise a thermoformed panel 393. In some embodiments, the adhesive is configured to provide mechanical pull through resilience. In some embodiments, the mechanical pull strength is configured to aid the electrical panel in returning to its original configuration after being bent. In some embodiments, electrical current is delivered to one or more electrodes through one or more traces, where the traces comprise printed electrically conductive material (e.g., ink). In some embodiments, the fingers 311 do not contain traces. In some embodiments, circuit material 320 comprises two or more circuit fingers 311 each extending in multiple directions a center of the terminal end of the circuit 320. In some embodiments, the shape of each of the fingers 311 allows flexing without bunching. In some embodiments, the shape includes an extended width, where a top view of one or more fingers includes a rectangular type of profile (e.g., a bar-like profile). In some embodiments, the two or more circuit fingers 311 are configured to deliver the electrical impulses to an electrode, heating element, and/or cooling element. In some embodiments, each electrode and/or element coupled to the electrical panel comprises two or more fingers 311.

In some embodiments, the front view 301 depicts circuit material 320 comprising circuit paths 330 connecting electrodes 381-383 represented by dashed boxes. In some embodiments, the flexible circuit 310 include one or more terminal holes 340 configured to interface with a terminal coupler 370 to provide a mechanical and/or electrical connection. In some embodiments, the one or more terminal holes 340 include ring terminal holes 340. In some embodiments, the flexible circuit 310 includes one or more ring terminal holes 340 in the flexible circuit configured to enable connection to a corresponding snap socket 370. In some embodiments, the snap socket 370 is installed via swaging or pressing, for example, and does not use soldering. In some embodiments, a rear post 371 of the snap socket 370 traps the flexible circuit and fabric together and provides both an electrical and mechanical connection. In some embodiments, the flexible circuit 310 comprises a dock 350 that curves to interface with a dock board 351 on the user side foam panel 391.

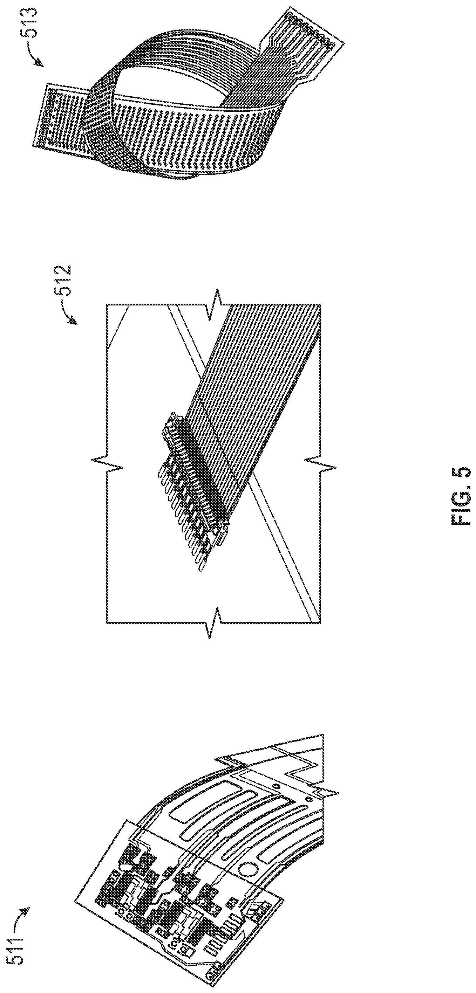

FIG. 4 depicts rendered views of the assembled electrical panel 303, as well as the thermoformed panel 393. FIG. 5 shows options for connecting the docket board to the flexible circuit as well as a rendering of the flexible circuit material 513. In some embodiments, a first option 511 for connecting the dock board includes fusing the dock board to the flexible circuit. In some embodiments, a second option 512 includes a dock board connection. In some embodiments, benefits of the system includes: no soldering, no sewing, no use of thermoplastic elastomers, low profile, flexes/contours on and around joints, provides structure/rigidity to electronics assembly supporting therapy application across multiple joints (on or around) through the modular electrical panel design and garments or bands.

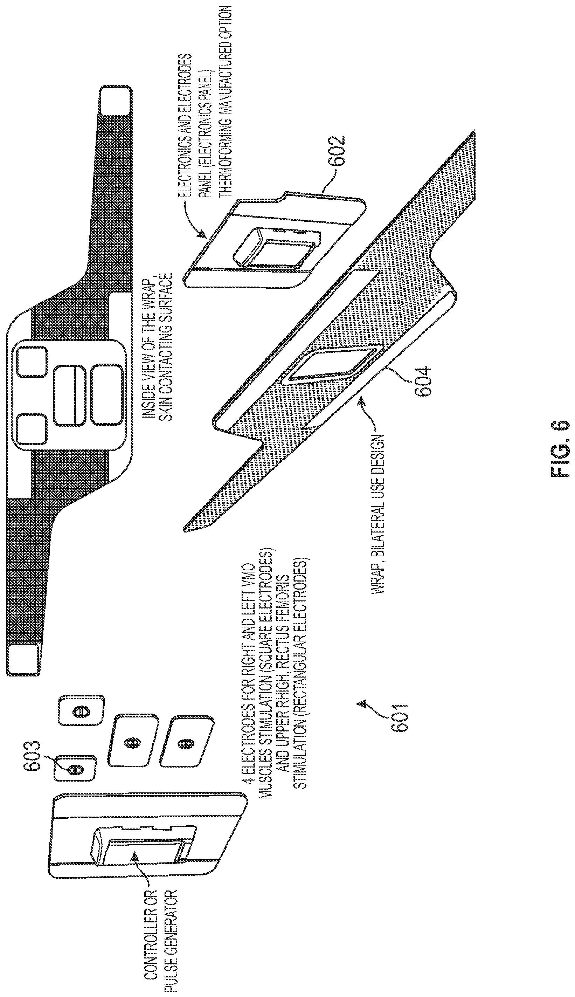

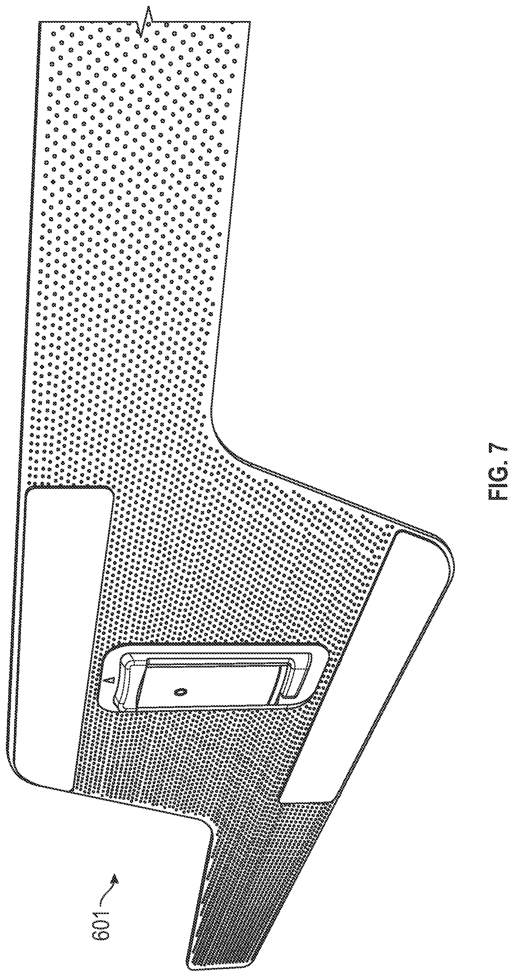

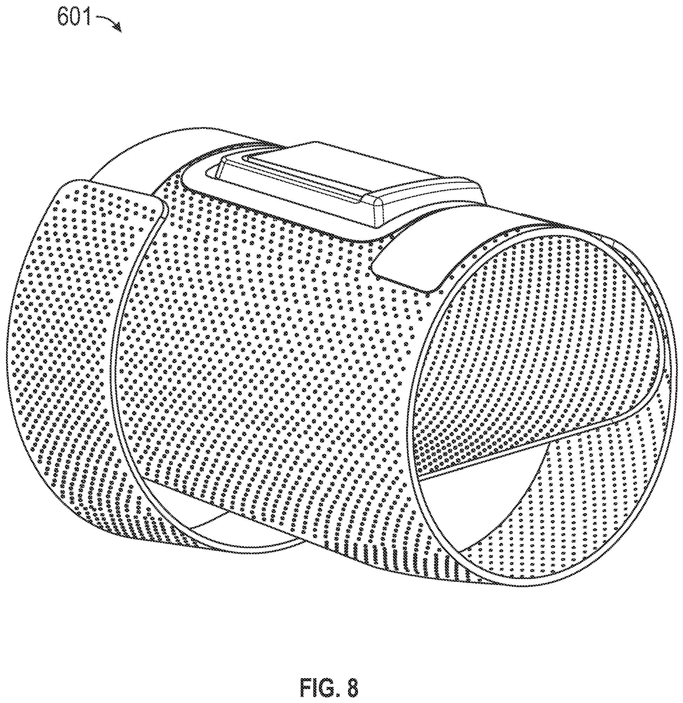

FIG. 6 depicts various components of the garment as a joint wrap 601 according to some embodiments. FIG. 7 shows a an assembled garment joint wrap 601 according to some embodiments. FIG. 8 illustrates how the joint wrap 601 is configured to secure around a user's joint according to some embodiments. In some embodiments, the garment comprises a modular system of a wrap or band and removable electrical panel including electronics and/or electrode assembly. In some embodiments, the garment comprises one or more of neoprene, Lycra foam, and/or other conventional flexible materials. In some embodiments, the garment is washable. In some embodiments, the garment comprises thermoformed edges for one or more of the wrap and the panel, which eliminates sewing operations. In some embodiments, the garment includes a die cut of the wrap and/or panel, eliminating sewing operations.

In some embodiments, the joint wrap system results in a simpler garment design and lighter weight. In some embodiments, the joint wrap 601 system is configured to provide mechanical support and/or compression to a user's joint. In some embodiments, the joint wrap includes an electrical panel 602 where the combination is configured to be a universal design where the same wrap and electrical panel combination can be used on a left or right limb. In some embodiments, the electrical panel 602 comprises a snap electrode interface 603. In some embodiments, the joint wrap system includes a modular system of a wrap and removable electrical panel 602 comprising electronics and/or an electrode assembly that includes one or more electrodes. In some embodiments, the system comprises a wrap and/or electrical panel 602 made from neoprene and/or Lycra foam type materials. In some embodiments, at least part of the wrap portion of the joint wrap system is washable, which is made possible by the modularity and removability of the electrical panel 602 and/or controller. In some embodiments, the electrical panel 602 includes an encapsulated foam molded panel design to hold and/or store the electronics. In some embodiments, electronics include one or more portions of electrical conductors (e.g., wires), electrodes, and/or controllers.

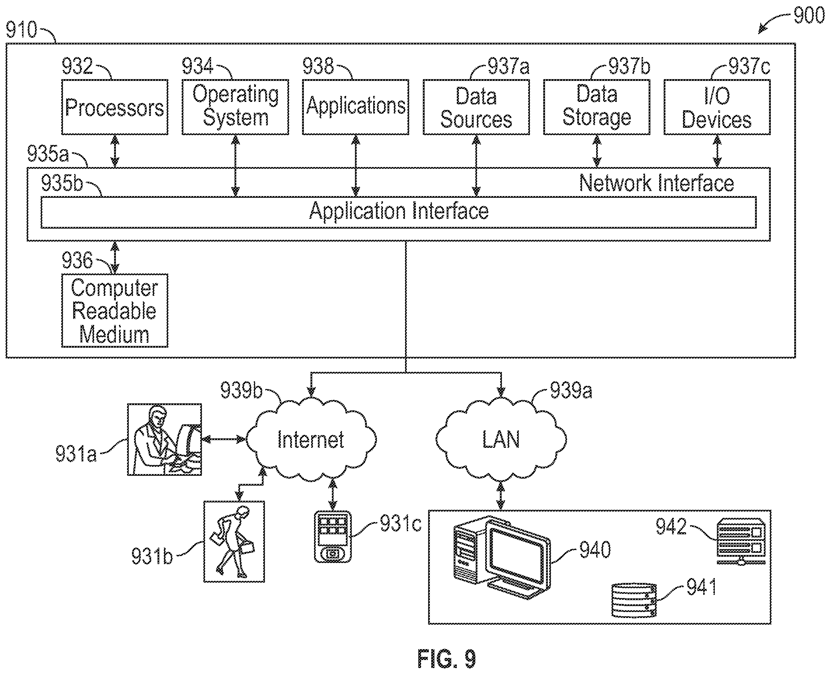

FIG. 9 illustrates a computer system 910 enabling or comprising the systems and methods in accordance with some embodiments of the system. In some embodiments, the computer system 910 is configured to operate and/or process computer-executable code of one or more software modules of the aforementioned system and method. Further, in some embodiments, the computer system 910 is configured to operate and/or display information within one or more graphical user interfaces (e.g., HMIs) integrated with or coupled to the system.

In some embodiments, the computer system 910 comprises one or more processors 932. In some embodiments, at least one processor 932 resides in, or is coupled to, one or more servers. In some embodiments, the computer system 910 includes a network interface 935a and an application interface 935b coupled to the least one processor 932 capable of processing at least one operating system 934. Further, in some embodiments, the interfaces 935a, 935b coupled to at least one processor 932 are configured to process one or more of the software modules (e.g., such as enterprise applications 938). In some embodiments, the software application modules 938 includes server-based software. In some embodiments, the software application modules 938 are configured to host at least one user account and/or at least one client account, and/or configured to operate to transfer data between one or more of these accounts using one or more processors 932.

With the above embodiments in mind, it is understood that the system is configured to implements various computer-implemented program steps involving data stored one or more non-transitory computer media according to some embodiments. In some embodiments, the above-described databases and models described throughout this disclosure are configured to store analytical models and other data on non-transitory computer-readable storage media within the computer system 910 and on computer-readable storage media coupled to the computer system 910 according to some embodiments. In addition, in some embodiments, the above-described applications of the system are stored on computer-readable storage media within the computer system 910 and on computer-readable storage media coupled to the computer system 910. In some embodiments, these operations are those requiring physical manipulation of quantities including electrons, electrical charges, transistors, amplifiers, receivers, transmitters, and/or any conventional computer hardware in order to transform an electrical input into a different output. In some embodiments, these quantities one or more of one or more of electrical, electromagnetic, magnetic, optical, or magneto-optical signals capable of being stored, transferred, combined, compared and otherwise manipulated. In some embodiments, the computer system 910 comprises at least one computer readable medium 936 coupled to at least one of at least one data source 937a, at least one data storage 937b, and/or at least one input/output 937c. In some embodiments, the computer system 910 is embodied as computer readable code on a computer readable medium 936. In some embodiments, the computer readable medium 936 includes any data storage that stores data, which is configured to thereafter be read by a computer (such as computer 940). In some embodiments, the non-transitory computer readable medium 936 includes any physical or material medium that is used to tangibly store the desired information, steps, and/or instructions and which is configured to be accessed by a computer 940 or processor 932. In some embodiments, the non-transitory computer readable medium 936 includes hard drives, network attached storage (NAS), read-only memory, random-access memory, FLASH based memory, CD-ROMs, CD-Rs, CD-RWs, DVDs, magnetic tapes, and/or other optical and non-optical data storage. In some embodiments, various other forms of computer-readable media 936 are configured to transmit or carry instructions to one or more remote computers 940 and/or at least one user 931, including a router, private or public network, or other transmission or channel, both wired and wireless. In some embodiments, the software application modules 938 are configured to send and receive data from a database (e.g., from a computer readable medium 936 including data sources 937a and data storage 937b that comprises a database), and data is configured to be received by the software application modules 938 from at least one other source. In some embodiments, at least one of the software application modules 938 are configured to be implemented by the computer system 910 to output data to at least one user 931 via at least one graphical user interface rendered on at least one digital display.

In some embodiments, the one or more non-transitory computer readable 936 media are distributed over a conventional computer network via the network interface 935a where some embodiments stored the non-transitory computer readable media are stored and executed in a distributed fashion. For example, in some embodiments, one or more components of the computer system 910 are configured to send and/or receive data through a local area network ("LAN") 939a and/or an internet coupled network 939b (e.g., such as a wireless internet). In some embodiments, the networks 939a, 939b include one or more wide area networks ("WAN"), direct connections (e.g., through a universal serial bus port), or other forms of computer-readable media 936, and/or any combination thereof.

In some embodiments, components of the networks 939a, 939b include any number of personal computers 940 which include for example desktop computers, laptop computers, and/or any fixed, generally non-mobile internet appliances coupled through the LAN 939a. For example, some embodiments include one or more personal computers 940, databases 941, and/or servers 942 coupled through the LAN 939a that are configured for use by any type of user including an administrator. Some embodiments include one or more personal computers 940 coupled through network 939b. In some embodiments, one or more components of the computer system 910 are configured to send or receive data through an internet network (e.g., such as network 939b). For example, some embodiments include at least one user 931a, 931b, coupled wirelessly and accessing one or more software modules of the system including at least one enterprise application 938 via an input and output ("I/O") 937c. In some embodiments, the computer system 910 is configured to enable at least one user 931a, 931b, to be coupled to access enterprise applications 938 via an I/O 937c through LAN 939a. In some embodiments, the user 931 includes a user 931a coupled to the computer system 910 using a desktop computer, and/or laptop computers, or any fixed, generally non-mobile internet appliances coupled through the internet 939b. In some embodiments, the user includes a mobile user 931b coupled to the computer system 910. In some embodiments, the user 931b connects using any mobile computing 931c to wireless coupled to the computer system 910, including, but not limited to, one or more personal digital assistants, at least one cellular phone, at least one mobile phone, at least one smart phone, at least one pager, at least one digital tablets, and/or at least one fixed or mobile internet appliances.

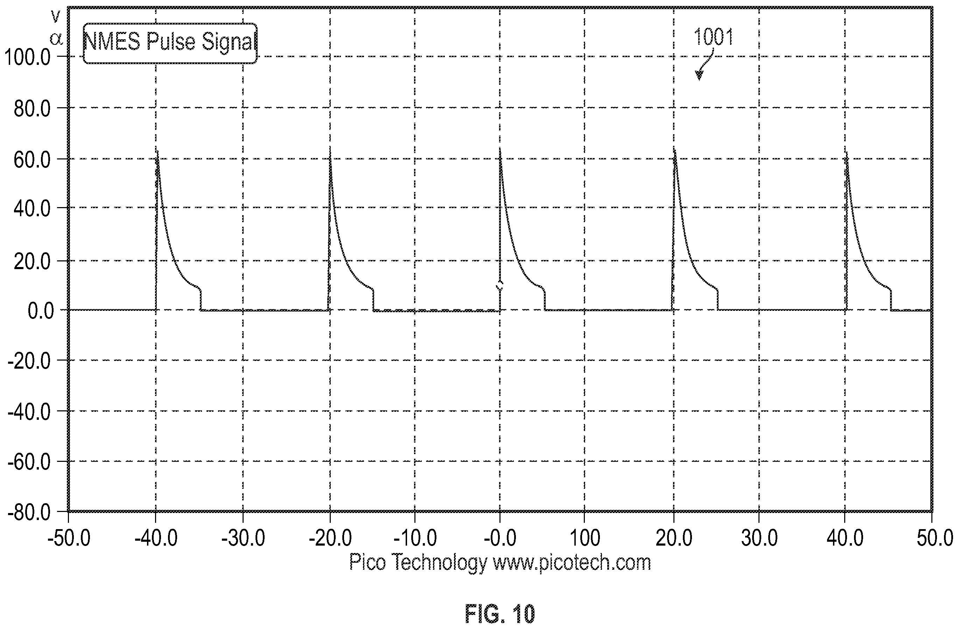

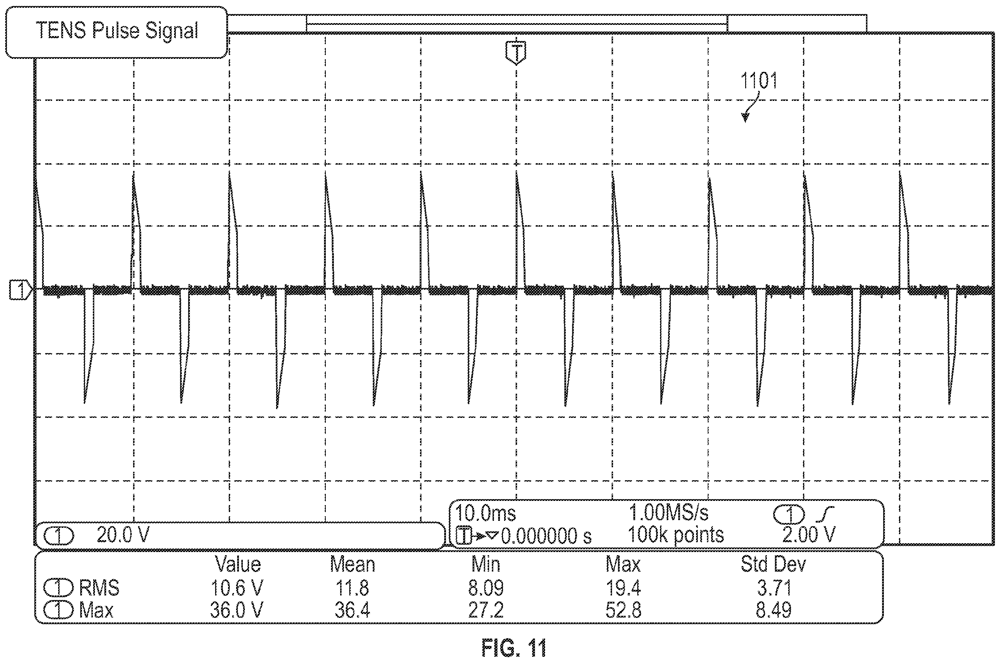

FIG. 10 depicts an NMES pulse signal profile 1001 executed by the system and implemented by one or more electrodes according to some embodiments. FIG. 11 shows a TENS pulse signal profile 1101 executed by the system and implemented by one or more electrodes according to some embodiments. In some embodiments, the system combines NMES and/or TENS therapies. In some embodiments, the system's pulse generator is configured to implement a NMES protocol with a TENS protocol in a single therapeutic session. In some embodiments, the NMES protocol includes program steps that provide the long-term benefits that include muscles strengthening, restoring muscle controls, and pain reduction by stimulating the motor neurons. In some embodiments, the TENS protocol includes program steps that provide the instant benefits of pain reduction by stimulating the sensory neurons, blocking the pain signals traveling from the brain to the joint. In some embodiments, the system applying a combination of the NMES and TENS provides both short-term and long-term pain reduction in a single therapy session, without extending therapy elapsed time, through interleaving NMES and TENS therapies.

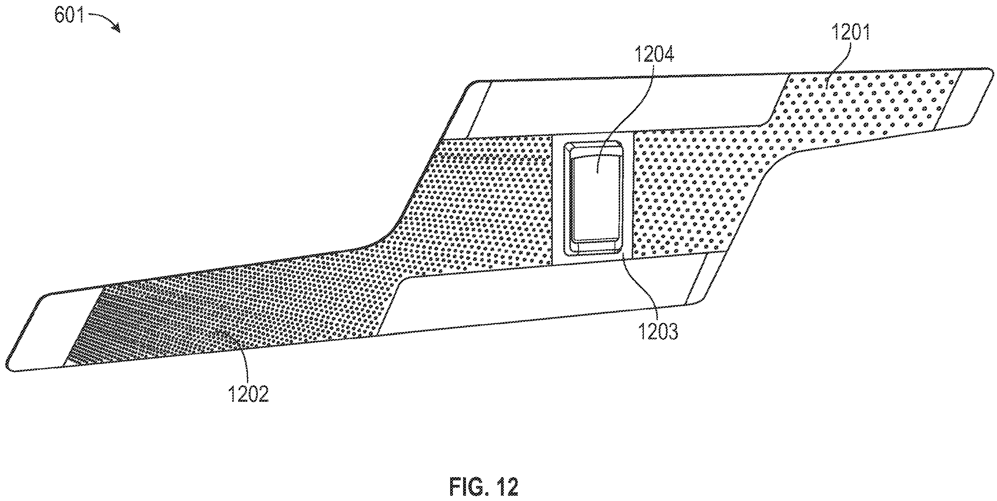

FIG. 12 shows a front view of some embodiments of the system that include a garment in the form of a joint wrap 601 according to some embodiments. In some embodiments, the joint wrap 601 comprises one or more flexible arms 1201, 1202 extending away from an electrical panel 602 mounting area 1203. In some embodiments, the flexible arms 1201, 1202 comprise flexible fabric. In some embodiments, the joint wrap 601 system comprises two flexible arms 1201, 1202 extending away from an electrical panel 602 mounting area 1203. In some embodiments, the two flexible arms 1201, 1202 comprise a first flexible fabric arm 1201 and a second flexible fabric arm 1202. In some embodiments, the first flexible arm 1201 extends away from a center 1204 of the panel mounting area 1203 a first distance. In some embodiments, the first flexible arm 1201 extends away from a center 1204 of the panel mounting area 1203 a second distance. In some embodiments, the first distance and the second distance comprise different lengths. In some embodiments, the first flexible arm 1201 comprises a shorter length than the second flexible arm 1202.

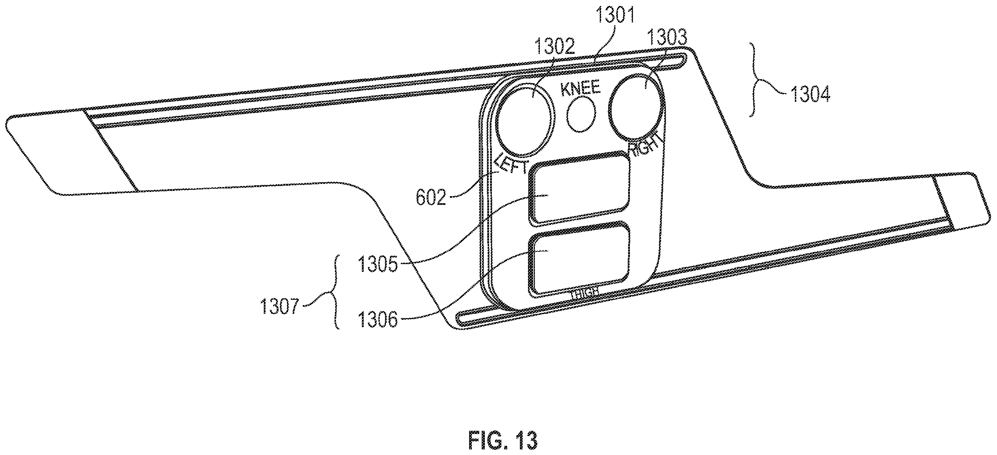

FIG. 13 shows a rear view of the wrap system according to some embodiments. In some embodiments, the rear view shows the electrical panel 602. In some embodiments, the joint wrap 601 comprises a panel mounting area 1203. In some embodiments, the panel mounting area comprises enough material to extend past an outer perimeter 1301 of the electrical panel 602.

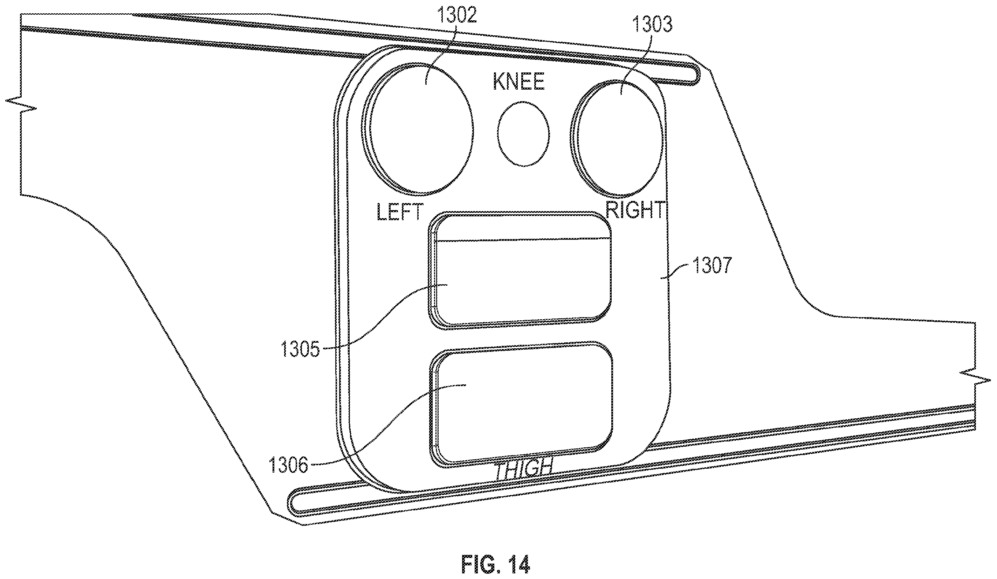

FIG. 14 is a closeup perspective view of FIG. 13 according to some embodiments. In some embodiments, the electrical panel 602 comprises a first side electrode 1302 and a second side electrode 1303. In some embodiments, both of the first side electrode 1302 and the second side electrode 1303 are both at least partially located in an upper portion 1304 of the electrical panel 602 when assembled to the joint wrap 601. In some embodiments, an upper portion 1304 of the electrical panel 602 includes an area of the joint wrap 601 where the first flexible arm 1201 extends away from the electrical panel 602. In some embodiments, the first side electrode 1302 and/or the second side electrode 1303 does not overlap a center portion 1204 of the electrical panel 602.

In some embodiments, the electrical panel 602 comprises one or more middle electrodes 1305. In some embodiments, the middle electrode 1305 at least partially overlaps a center 1204 of the electrical panel 602. In some embodiments, the middle electrode 1305 is located at least partially between the first flexible arm 1201 and the second flexible arm 1201.

In some embodiments, the electrical panel 602 comprises one or more lower electrodes 1306. In some embodiments, the lower electrode 1306 is at least partially located in a lower portion 1307 of the electrical panel 602 when assembled to the joint wrap 601. In some embodiments, a lower portion 1307 of the electrical panel 602 includes an area of the joint wrap 601 where the second flexible arm 1202 extends away from the electrical panel 602. In some embodiments, the lower electrode 1306 does not overlap a center portion 1204 of the electrical panel 602.

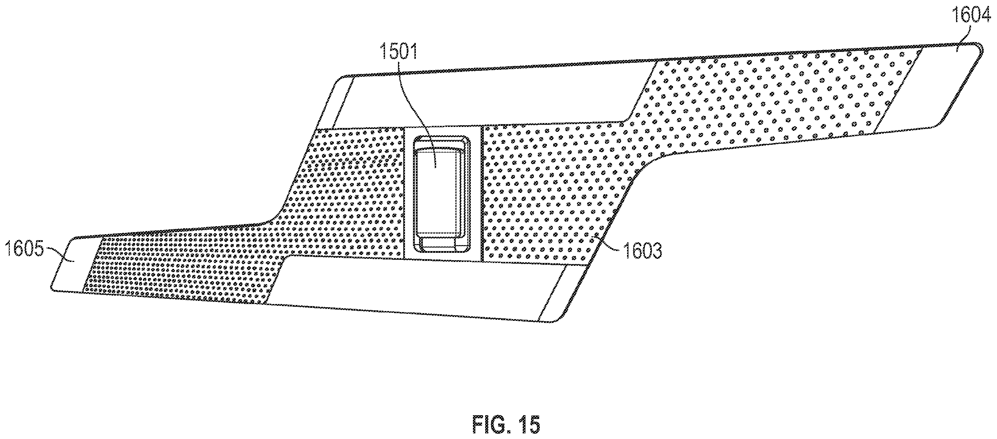

FIG. 15 is another perspective view of FIG. 12 according to some embodiments. In some embodiments, the electrical panel 602 comprises a removable controller 1501. In some embodiments, the removable controller 1501 is positioned on an opposite side of the joint wrap 601 than one or more electrodes previously described. In some embodiments, the controller 1501 comprises at least one battery configured to store electrical energy. In some embodiments, the controller 1501 comprises a wire power input configured to deliver electrical energy to the at least one battery and/or the controller 1501. In some embodiments, the controller 1501 at least partially overlaps a center portion 1204 of the electrical panel 602 when assembled. In some embodiments, the controller 1501 is configured to removably couple to the electrical panel 602 through one or more fasteners configured to be released by hand.

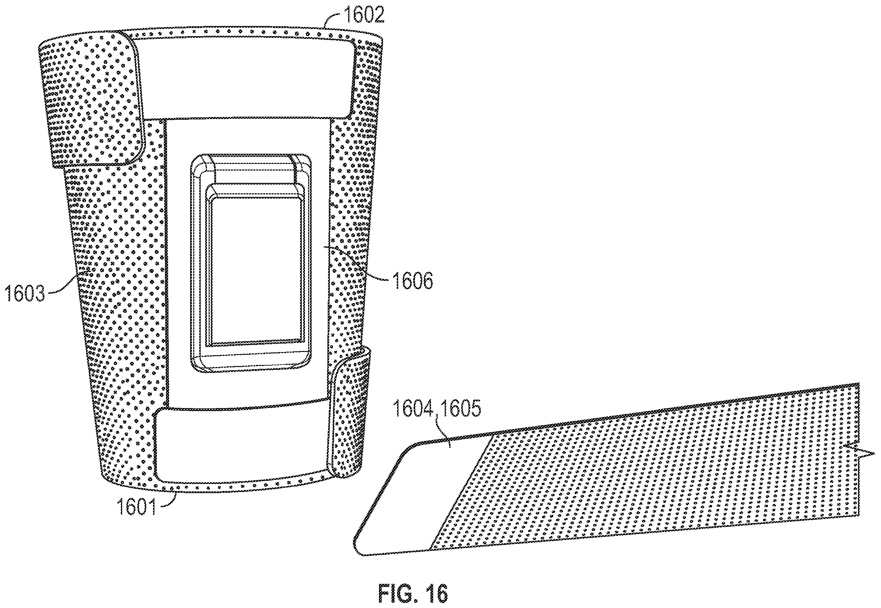

FIG. 16 illustrates a wrapped view of the system according to some embodiments. In some embodiments, the different lengths of the first flexible arm 1201 and the second flexible arm 1202 are configured to create a first circumference 1601 and a second circumference 1602 when removably coupled to the joint wrap 601 main body 1603. In some embodiments, the first circumference 1601 and the second circumference 1602 are different circumferences when an equal end area 1604, 1605 of each of the first flexible arm 1201 and second flexible arm 1202 overlap the joint wrap 601 main body 1603. In some embodiments, this provides the benefit of better securing to an upper and lower portion of a limb, while minimizing material needed for production.

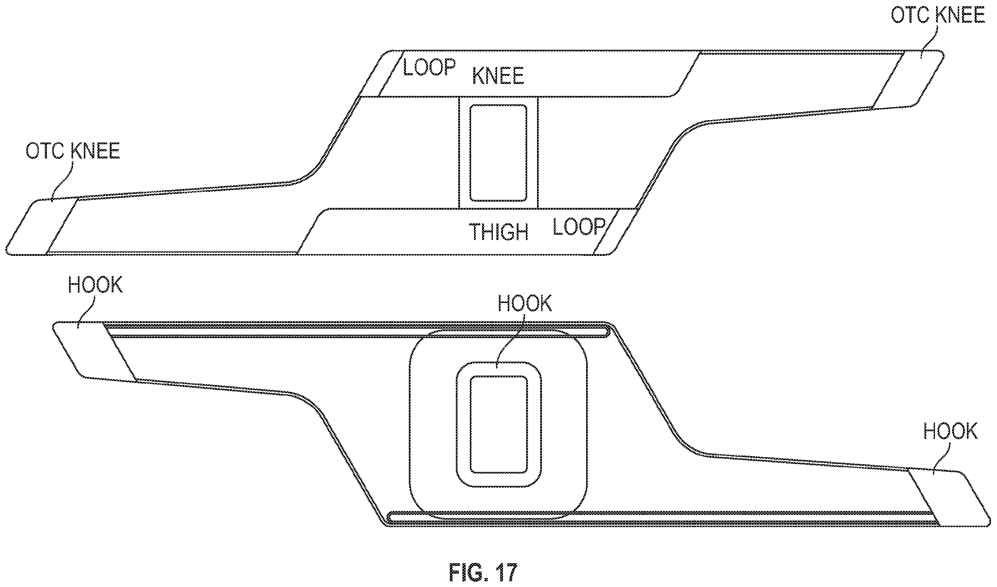

FIG. 17 is a line drawing with various labels describing aspects of FIGs. 12 and 13 according to some embodiments. In some embodiments, the line drawing shows a heat-pressed edge 1701, a knee placement area 1702, a thigh placement area 1703, a first flexible arm 1201, first main body coupler 1704, a second flexible arm 1202, second main body coupler 1705, a hollow controller area 1706, an electrical panel coupler 1707, as well as various non-limiting dimensions according to some embodiments. In some embodiments, the hollow controller area 1706 provides the benefit of allowing at least a portion of the electrical panel 602 to pass through the joint wrap 601. In some embodiments, the hollow controller area 1706 provides the benefit of allowing at least a portion of the controller 1501 to pass through the joint wrap 601.

In some embodiments, the first main body coupler 1705 is configured to securely couple to a first arm coupler 1708 located at a first end area 1604. In some embodiments, the second main body coupler 1705 is configured to securely couple to a second arm coupler 1709 located at a second end area 1605. In some embodiments, at least a portion of the electrical panel 602 is configured to couple to a panel coupler 1707 to secure the electrical panel 602 in one or more positions. In some embodiments, the panel coupler 1707, as well as any coupler described herein, comprises a hook, Velcro^{®}, snap fitting, or one or more conventional fasteners. In some embodiments, the panel coupler 1707 includes a coupling plate 1606 comprising a larger area than the main body hollow portion 1706. In some embodiments, coupling plate 1606 is configured to be placed on an opposite side of the main body 1603 than the electrodes 1302-1306. In some embodiments, the coupling plate 1606 is configured to couple to the electrical panel 602, where the larger area prevents decoupling of the electrical panel 602 from the main body 1603 by preventing the coupling plate 1606 from passing through the hollow portion 1706.

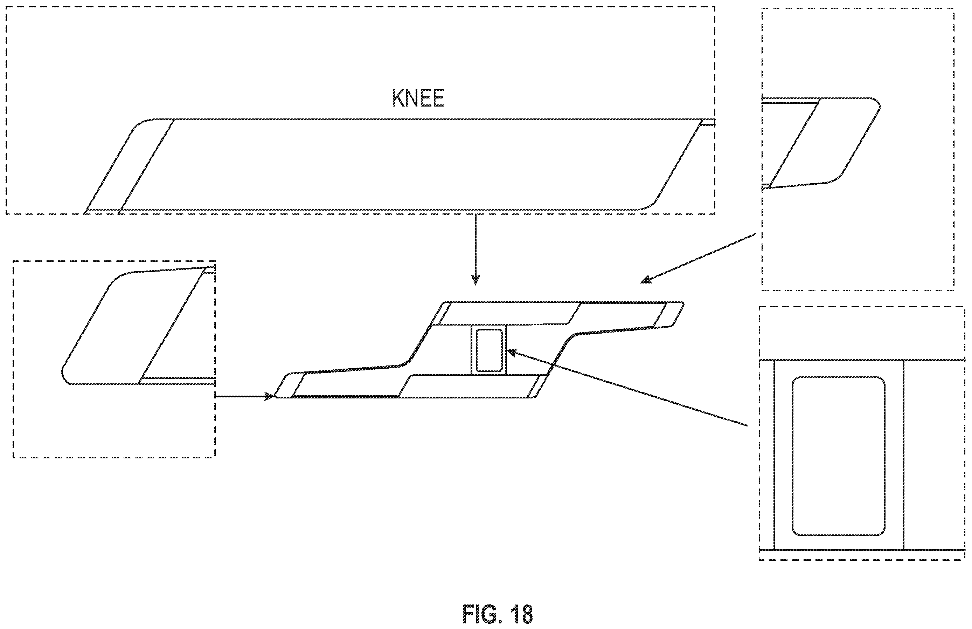

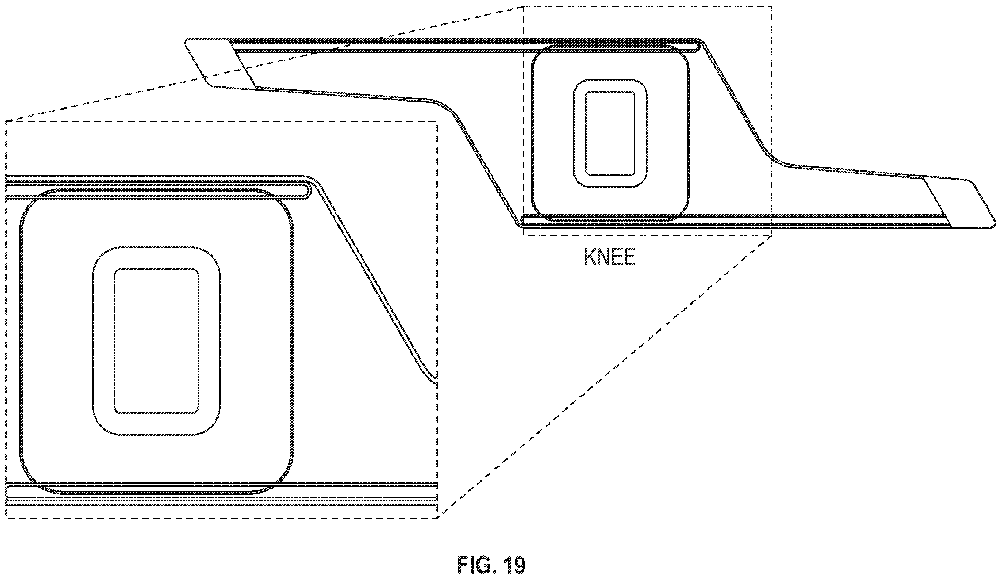

FIG. 18 shows various dimensions of FIG. 12 according to some embodiments. FIG. 19 depicts further various dimensions of the wrap system of FIG. 13 according to some embodiments. The example dimensions are in no way limiting and are merely shown to aid those of ordinary skill to make and user the system.

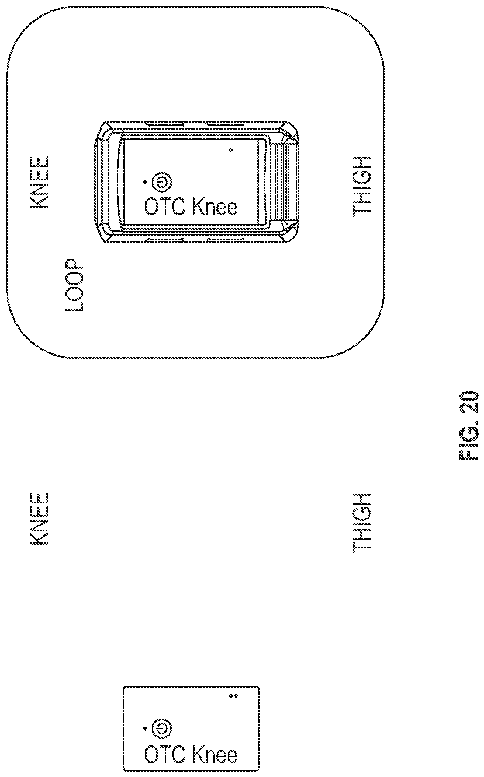

FIG. 20 shows various aspects of a front portion of the electrical panel 602 according to some embodiments. In some embodiments, at least a portion of the controller 1501 is configured to pass through a panel hole 604 located in a center portion of the main body 1603. In some embodiments, the electrical panel 602 comprises the controller 1501. In some embodiments, the controller 1501 and electrical panel 602 are integrally coupled. In some embodiments, the electrical panel 602 is configured to couple to the integrally coupled controller 1501 to prevent the electrical panel 602 from passing through the hollow portion 1706 of the main body 1603.

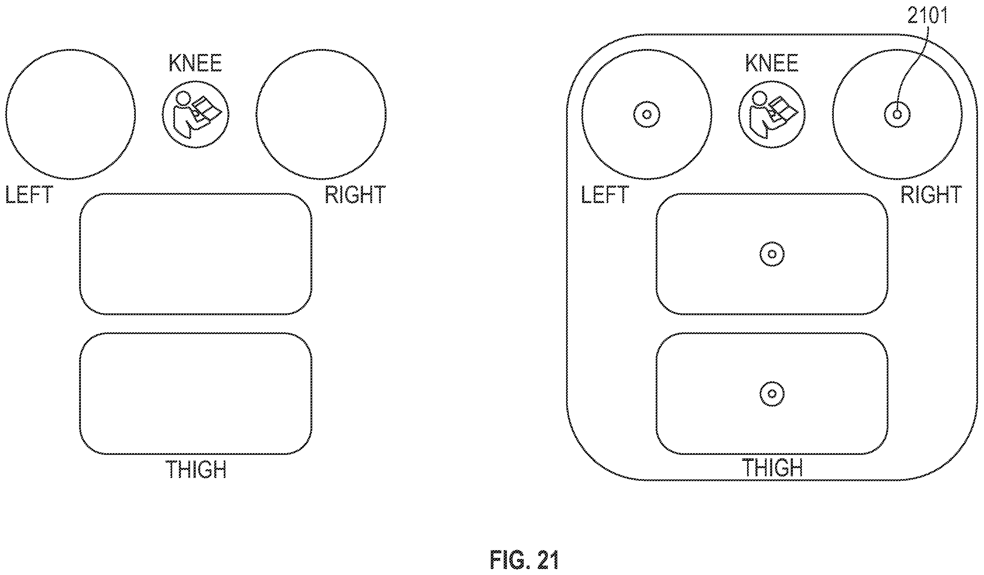

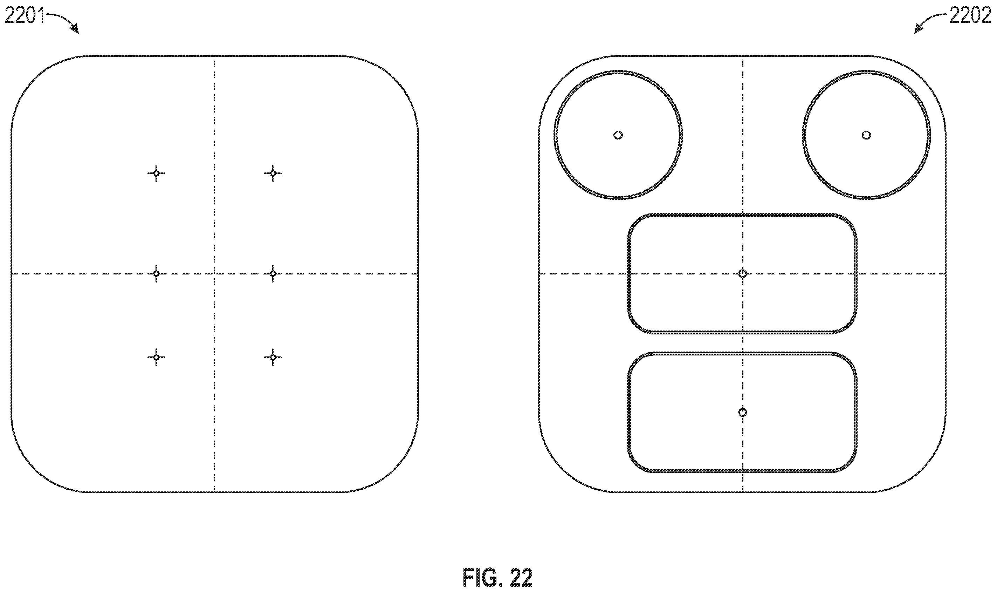

FIG. 21 shows various aspects of a rear portion of the electrical panel 602 according to some embodiments. In some embodiments, the electrical panel 602 comprises one or more electrodes 1302-1306. In some embodiments, the electrical panel 602 comprise one or more electrode contacts 2101. In some embodiments, the one or more electrode contacts 2101 are configured to enable both mechanical coupling and electrical connectivity to an electrode 1302-1306. In some embodiments, one or more of the one or more electrode contacts 2101 are each configured to enable decoupling of the electrode 1302-1306 from the electrical panel 602. This provides the benefit of being able to replace worn electrodes according to some embodiments. FIG. 22 shows various non-limiting outlines and dimensions of the front and rear portions of the electrical panel 602 according to some embodiments.

The subject matter described herein are directed to technological improvements to the field of pain mitigation though new arrangements and applications of NMES or other neuromodulation therapies or thermotherapy methods. The disclosure describes the specifics of how a machine including one or more computers comprising one or more processors and one or more non-transitory computer readable media implement the system and its improvements over the prior art. The instructions executed by the machine cannot be performed in the human mind or derived by a human using a pen and paper but require the machine to convert process input data to useful output data. Moreover, the claims presented herein do not attempt to tie-up a judicial exception with known conventional steps implemented by a general-purpose computer; nor do they attempt to tie-up a judicial exception by simply linking it to a technological field. Indeed, the systems and methods described herein were unknown and/or not present in the public domain at the time of filing, and they provide technologic improvements advantages not known in the prior art. Furthermore, the system includes unconventional steps that confine the claim to a useful application.

It is understood that the system is not limited in its application to the details of construction and the arrangement of components set forth in the previous description or illustrated in the drawings. The system and methods disclosed herein fall within the scope of numerous embodiments. The previous discussion is presented to enable a person skilled in the art to make and use embodiments of the system. Any portion of the structures and/or principles included in some embodiments can be applied to any and/or all embodiments: it is understood that features from some embodiments presented herein are combinable with other features according to some other embodiments. Thus, some embodiments of the system are not intended to be limited to what is illustrated but are to be accorded the widest scope consistent with all principles and features disclosed herein.

Some embodiments of the system are presented with specific values and/or setpoints. These values and setpoints are not intended to be limiting and are merely examples of a higher configuration versus a lower configuration and are intended as an aid for those of ordinary skill to make and use the system.

Furthermore, acting as Applicant's own lexicographer, Applicant imparts the explicit meaning and/or disavow of claim scope to the following terms:

Applicant defines any use of "and/or" such as, for example, "A and/or B," or "at least one of A and/or B" to mean element A alone, element B alone, or elements A and B together. In addition, a recitation of "at least one of A, B, and C," a recitation of "at least one of A, B, or C," or a recitation of "at least one of A, B, or C or any combination thereof" are each defined to mean element A alone, element B alone, element C alone, or any combination of elements A, B and C, such as AB, AC, BC, or ABC, for example.

"Substantially" and "approximately" when used in conjunction with a value encompass a difference of 5% or less of the same unit and/or scale of that being measured.

"Simultaneously" as used herein includes lag and/or latency times associated with a conventional and/or proprietary computer, such as processors and/or networks described herein attempting to process multiple types of data at the same time. "Simultaneously" also includes the time it takes for digital signals to transfer from one physical location to another, be it over a wireless and/or wired network, and/or within processor circuitry.

As used herein, "can" or "may" or derivations there of (e.g., the system display can show X) are used for descriptive purposes only and is understood to be synonymous and/or interchangeable with "configured to" (e.g., the computer is configured to execute instructions X) when defining the metes and bounds of the system.

In addition, the term "configured to" means that the limitations recited in the specification and/or the claims must be arranged in such a way to perform the recited function: "configured to" excludes structures in the art that are "capable of" being modified to perform the recited function but the disclosures associated with the art have no explicit teachings to do so. For example, a recitation of a "container configured to receive a fluid from structure X at an upper portion and deliver fluid from a lower portion to structure Y" is limited to systems where structure X, structure Y, and the container are all disclosed as arranged to perform the recited function. The recitation "configured to" excludes elements that may be "capable of" performing the recited function simply by virtue of their construction but associated disclosures (or lack thereof) provide no teachings to make such a modification to meet the functional limitations between all structures recited. Another example is "a computer system configured to or programmed to execute a series of instructions X, Y, and Z." In this example, the instructions must be present on a non-transitory computer readable medium such that the computer system is "configured to" and/or "programmed to" execute the recited instructions: "configure to" and/or "programmed to" excludes art teaching computer systems with non-transitory computer readable media merely "capable of" having the recited instructions stored thereon but have no teachings of the instructions X, Y, and Z programmed and stored thereon. The recitation "configured to" can also be interpreted as synonymous with operatively connected when used in conjunction with physical structures.

It is understood that the phraseology and terminology used herein is for description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

The previous detailed description is to be read with reference to the figures, in which like elements in different figures have like reference numerals. The figures, which are not necessarily to scale, depict some embodiments and are not intended to limit the scope of embodiments of the system.

Any of the operations described herein that form part of the invention are useful machine operations. The invention also relates to a device or an apparatus for performing these operations. The apparatus can be specially constructed for the required purpose, such as a special purpose computer. When defined as a special purpose computer, the computer can also perform other processing, program execution or routines that are not part of the special purpose, while still being capable of operating for the special purpose. Alternatively, the operations can be processed by a general-purpose computer selectively activated or configured by one or more computer programs stored in the computer memory, cache, or obtained over a network. When data is obtained over a network the data can be processed by other computers on the network, e.g. a cloud of computing resources.

The embodiments of the invention can also be defined as a machine that transforms data from one state to another state. The data can represent an article, that can be represented as an electronic signal and electronically manipulate data. The transformed data can, in some cases, be visually depicted on a display, representing the physical object that results from the transformation of data. The transformed data can be saved to storage generally, or in particular formats that enable the construction or depiction of a physical and tangible object. In some embodiments, the manipulation can be performed by a processor. In such an example, the processor thus transforms the data from one thing to another. Still further, some embodiments include methods can be processed by one or more machines or processors that can be connected over a network. Each machine can transform data from one state or thing to another, and can also process data, save data to storage, transmit data over a network, display the result, or communicate the result to another machine. Computer-readable storage media, as used herein, refers to physical or tangible storage (as opposed to signals) and includes without limitation volatile and non-volatile, removable and non-removable storage media implemented in any method or technology for the tangible storage of information such as computer-readable instructions, data structures, program modules or other data.

Although method operations are presented in a specific order according to some embodiments, the execution of those steps do not necessarily occur in the order listed unless explicitly specified. Also, other housekeeping operations can be performed in between operations, operations can be adjusted so that they occur at slightly different times, and/or operations can be distributed in a system which allows the occurrence of the processing operations at various intervals associated with the processing, as long as the processing of the overlay operations are performed in the desired way and result in the desired system output.

Various features and advantages of the invention are set forth in the following claims.

## Claims

1. A system (201) comprising:
a garment (101),
one or more electrodes (381-383),
at least one controller (1501); and
an electrical panel (602);
wherein the garment (101) is configured to wrap around a biological joint;
wherein the one or more electrodes (381-383) are coupled to the garment (101);
wherein the at least one controller (1501) is coupled to the one or more electrodes (381-383);
wherein the at least one controller (1501) is configured to send one or more electrical pulses to the one or more electrodes (381-383) while the garment (101) is wrapped around the biological joint; and
wherein the one or more electrical pulses are configured to stimulate one or more of a nerve
and a muscle in an area of the biological joint;
**characterized in that**
wherein the one or more electrodes (381-383) are coupled to the electrical panel (602) via one or more flexible fingers;
wherein the electrical panel (602) is coupled to the garment (101); and
wherein the one or more flexible fingers are configured to prevent a bunching of the electrical panel (602) in an area of the one or more flexible fingers when the electrical panel (602) is deformed with a deformation of the garment (101).

2. The system of claim 1,
wherein the electrical panel (602) comprises an electrical circuit (310); and
wherein the electrical circuit (310) is configured to transmit the one or more electrical pulses to the one or more electrodes (381-383).

3. The system of claim 2,
wherein the electrical panel (602) is a flexible electrical panel; and
wherein the flexible electrical panel is configured to at least partially conform to a shape of the garment (101) when the garment (101) is deformed during a wrapping around the biological joint.

4. The system of claim 3,
wherein the electrical panel (602) is configured to be detachably removed from the garment (101).

5. The system of claim 4,
wherein at least a portion of the electrical panel (602) is configured to pass through a hollow portion (1706) of the garment (101).

6. The system of claim 4,
wherein the electrical panel (602) comprises at least one controller coupling;
wherein the at least one controller (1501) coupling is configured to surround at least a portion of the at least one controller (1501); and
wherein the at least one controller (1501) is configured to be detachably coupled to the at least one controller coupling.

7. The system of claim 6,
wherein the garment (101) comprises a main body (1603) and one or more arms (1201,1202);
wherein the one or more arms (1201,1202) extend away from the main body (1603);
wherein the main body (1603) is configured and arranged to extend past a parameter of the electrical panel (602);
wherein the main body (1603) comprises a hollow portion (1706); and
wherein at least one of the at least one controller (1501) and the at least one controller coupling are configured to pass through the hollow portion (1706) of the main body (1603) when the electrical panel (602) is coupled to the main body (1603)
wherein the one or more arms (1201,1202) are preferably two arms (1201,1202);
wherein preferably a first arm (1201) of the two arms (1201,1202) comprises a first length;
wherein preferably a second arm (1202) of the two arms (1201,1202) comprises a second length; and
wherein preferably the first length is different from the second length.

8. The system of claim 7,
wherein the electrical circuit (310) is a flexible electrical circuit;
wherein the flexible electrical circuit includes one or more electrode couplings; and
wherein the one or more electrode couplings are each configured to enable removable electrical coupling of each of the one or more electrodes (381-383).

9. The system of claim 8,
wherein each of the one or more electrode couplings comprise the one or more flexible fingers extending from a center of the one or more electrode couplings.

10. The system of claim 1,
wherein the flexible electrical circuit comprises one or more electricity flow paths;
wherein the one or more electricity flow paths are configured to deliver electricity from the at least one controller (1501) to the one or more electrode couplings; and
wherein at the one or more electricity flow paths are configured to extend along a finger length of at least one of the one or more flexible fingers at each of the one or more electrode couplings
wherein the flexible electrical circuit, the one or more electricity flow paths, and the one or more flexible fingers are preferably housed within an internal portion of the electrical panel.

## Patentansprüche

1. System (201), umfassend:
ein Textil (101),
mindestens eine Elektrode (381-383),
mindestens eine Steuereinheit (1501); und
ein elektrisches Panel (602);
wobei das Textil (101) dazu eingerichtet ist, sich um ein biologisches Gelenk zu wickeln;
wobei die mindestens eine Elektrode (381-383) mit dem Textil (101) gekoppelt ist;
wobei die mindestens eine Steuereinheit (1501) mit der mindestens einen Elektrode (381-383) gekoppelt ist;
wobei die mindestens eine Steuereinheit (1501) dazu eingerichtet ist, mindestens einen elektrischen Impuls an die mindestens eine Elektrode (381-383) zu senden, während das Textil (101) um das biologische Gelenk gewickelt ist; und
wobei der mindestens eine elektrische Impuls dazu eingerichtet ist, eines oder mehrere aus einem Nerv und einem Muskel in einem Bereich des biologischen Gelenks zu stimulieren,
**dadurch gekennzeichnet, dass**
die mindestens eine Elektrode (381-383) über mindestens einen flexiblen Finger mit dem elektrischen Panel (602) gekoppelt ist;
wobei das elektrische Panel (602) mit dem Textil (101) gekoppelt ist; und
wobei der mindestens eine flexible Finger dazu eingerichtet ist, eine Zusammenballung des elektrischen Panels (602) in einem Bereich des mindestens einen flexiblen Fingers zu verhindern, wenn das elektrische Panel (602) durch eine Verformung des Textils (101) verformt wird.

2. System nach Anspruch 1,
wobei das elektrische Panel (602) eine elektrische Schaltung (310) umfasst; und
wobei die elektrische Schaltung (310) dazu eingerichtet ist, den mindestens einen elektrischen Impuls an die mindestens eine Elektrode (381-383) zu übertragen.

3. System nach Anspruch 2,
wobei das elektrische Panel (602) ein flexibles elektrisches Panel ist; und
wobei das flexible elektrische Panel dazu eingerichtet ist, sich zumindest teilweise an eine Form des Textils (101) anzupassen, wenn das Textil (101) beim Wickeln um das biologische Gelenk verformt wird.

4. System nach Anspruch 3,
wobei das elektrische Panel (602) dazu eingerichtet ist, lösbar von dem Textil (101) abgenommen zu werden.

5. System nach Anspruch 4,
wobei zumindest ein Teil des elektrischen Panels (602) dazu eingerichtet ist, durch einen hohlen Abschnitt (1706) des Textils (101) hindurch zu verlaufen.

6. System nach Anspruch 4,
wobei das elektrische Panel (602) mindestens eine Steuereinheitskopplung umfasst;
wobei die mindestens eine Steuereinheitskopplung dazu eingerichtet ist, zumindest einen Teil der mindestens einen Steuereinheit (1501) zu umgeben; und
wobei die mindestens eine Steuereinheit (1501) dazu eingerichtet ist, lösbar mit der mindestens einen Steuereinheitskopplung gekoppelt zu sein.

7. System nach Anspruch 6,
wobei das Textil (101) einen Hauptkörper (1603) und mindestens einen Arm (1201, 1202) umfasst;
wobei sich der mindestens eine Arm (1201, 1202) von dem Hauptkörper (1603) weg erstreckt;
wobei der Hauptkörper (1603) dazu eingerichtet und angeordnet ist, sich über einen Umfang des elektrischen Panels (602) hinaus zu erstrecken;
wobei der Hauptkörper (1603) einen hohlen Abschnitt (1706) umfasst; und
wobei mindestens eines von der mindestens einen Steuereinheit (1501) und der mindestens einen Steuereinheitskopplung dazu eingerichtet ist, durch den hohlen Abschnitt (1706) des Hauptkörpers (1603) hindurch zu verlaufen, wenn das elektrische Panel (602) mit dem Hauptkörper (1603) gekoppelt ist;
wobei der mindestens eine Arm (1201, 1202) vorzugsweise zwei Arme (1201, 1202) sind;
wobei ein erster Arm (1201) der zwei Arme (1201, 1202) vorzugsweise eine erste Länge aufweist;
wobei ein zweiter Arm (1202) der zwei Arme (1201, 1202) vorzugsweise eine zweite Länge aufweist; und
wobei sich die erste Länge vorzugsweise von der zweiten Länge unterscheidet.

8. System nach Anspruch 7,
wobei die elektrische Schaltung (310) eine flexible elektrische Schaltung ist;
wobei die flexible elektrische Schaltung mindestens eine Elektrodenkopplung umfasst; und
wobei die mindestens einen Elektrodenkopplung jeweils dazu eingerichtet ist, eine lösbare elektrische Kopplung von jeder der mindestens einen Elektrode (381-383) zu ermöglichen.

9. System nach Anspruch 8,
wobei jede der mindestens einen Elektrodenkopplung den mindestens einen flexiblen Finger umfasst, der sich von einem Zentrum der mindestens einen Elektrodenkopplung erstreckt.

10. System nach Anspruch 1,
wobei die flexible elektrische Schaltung mindestens einen Strompfad umfasst;
wobei der mindestens eine Strompfad dazu eingerichtet ist, elektrische Energie von der mindestens einen Steuereinheit (1501) zu der mindestens einen Elektrodenkopplung zu übertragen; und
wobei sich der mindestens eine Strompfad an jeder der mindestens einen Elektrodenkopplung entlang einer Fingerlänge mindestens eines der mindestens einen flexiblen Finger erstreckt,
wobei die flexible elektrische Schaltung, der mindestens eine Strompfad und der mindestens eine flexible Finger vorzugsweise innerhalb eines Innenabschnitts des elektrischen Panels aufgenommen sind.

## Revendications

1. Système (201) comprenant :
un vêtement (101),
une ou plusieurs électrodes (381-383),
au moins un dispositif de commande (1501) ; et
un panneau électrique (602) ;
dans lequel le vêtement (101) est configuré pour s'enrouler autour d'une articulation biologique ;
dans lequel les une ou plusieurs électrodes (381-383) sont raccordées au vêtement (101) ;
dans lequel l'au moins un dispositif de commande (1501) est raccordé aux une ou plusieurs électrodes (381-383) ; dans lequel l'au moins un dispositif de commande (1501) est configuré pour envoyer une ou plusieurs impulsions électriques aux une ou plusieurs électrodes (381-383) tandis que le vêtement (101) est enroulé autour de l'articulation biologique ; et
dans lequel les une ou plusieurs impulsions électriques sont configurées pour stimuler un ou plusieurs d'un nerf et d'un muscle dans une zone de l'articulation biologique ;
**caractérisé en ce que**
dans lequel les une ou plusieurs électrodes (381-383) sont raccordées au panneau électrique (602) par l'intermédiaire d'un ou plusieurs doigts flexibles ;
dans lequel le panneau électrique (602) est raccordé au vêtement (101) ; et
dans lequel les un ou plusieurs doigts flexibles sont configurés pour empêcher un plissement du panneau électrique (602) dans une zone des un ou plusieurs doigts flexibles lorsque le panneau électrique (602) est déformé avec une déformation du vêtement (101).

2. Système selon la revendication 1,
dans lequel le panneau électrique (602) comprend un circuit électrique (310) ; et
dans lequel le circuit électrique (310) est configuré pour transmettre les une ou plusieurs impulsions électriques aux une ou plusieurs électrodes (381-383).

3. Système selon la revendication 2,
dans lequel le panneau électrique (602) est un panneau électrique flexible ; et
dans lequel le panneau électrique flexible est configuré pour se conformer au moins partiellement à une forme du vêtement (101) lorsque le vêtement (101) est déformé pendant un enroulement autour de l'articulation biologique.

4. Système selon la revendication 3,
dans lequel le panneau électrique (602) est configuré pour être retiré de manière détachable du vêtement (101).

5. Système selon la revendication 4,
dans lequel au moins une partie du panneau électrique (602) est configurée pour passer à travers une partie creuse (1706) du vêtement (101).

6. Système selon la revendication 4,
dans lequel le panneau électrique (602) comprend au moins un raccord de dispositif de commande ;
dans lequel l'au moins un raccord de dispositif de commande (1501) est configuré pour entourer au moins une partie de l'au moins un dispositif de commande (1501) ; et
dans lequel l'au moins dispositif de commande (1501) est configuré pour être raccordé de manière détachable à l'au moins un raccord de dispositif de commande.

7. Système selon la revendication 6,
dans lequel le vêtement (101) comprend un corps principal (1603) et un ou plusieurs bras (1201, 1202) ;
dans lequel les un ou plusieurs bras (1201, 1202) s'étendent à distance du corps principal (1603) ;
dans lequel le corps principal (1603) est configuré et agencé pour s'étendre au-delà d'un paramètre du panneau électrique (602) ;
dans lequel le corps principal (1603) comprend une partie creuse (1706) ; et
dans lequel au moins l'un parmi l'au moins un dispositif de commande (1501) et l'au moins un raccord de dispositif de commande sont configurés pour passer à travers la partie creuse (1706) du corps principal (1603) lorsque le panneau électrique (602) est raccordé au corps principal (1603)
dans lequel les un ou plusieurs bras (1201, 1202) sont de préférence deux bras (1201, 1202) ;
dans lequel un premier bras (1201) des deux bras (1201, 1202) a une première longueur ;
dans lequel un deuxième bras (1202) des deux bras (1201, 1202) a une deuxième longueur ; et
dans lequel la première longueur est de préférence différente de la deuxième longueur.

8. Système selon la revendication 7,
dans lequel le circuit électrique (310) est un circuit électrique flexible ;
dans lequel le circuit électrique flexible comprend un ou plusieurs raccords d'électrodes ; et
dans lequel les un ou plusieurs raccords d'électrodes sont chacun configurés pour permettre un raccordement électrique détachable de chacune des une ou plusieurs électrodes (381-383).

9. Système selon la revendication 8,
dans lequel chacun des un ou plusieurs raccords d'électrodes comprend les un ou plusieurs doigts flexibles s'étendant à partir d'un centre des un ou plusieurs raccords d'électrode.

10. Système selon la revendication 1,
dans lequel le circuit électrique flexible comprend un ou plusieurs chemins d'écoulement de l'électricité ;
dans lequel les un ou plusieurs chemins d'écoulement de l'électricité sont configurés pour fournir de l'électricité à partir de l'au moins un dispositif de commande (1501) aux un ou plusieurs raccords d'électrodes ; et
dans lequel les un ou plusieurs chemins d'écoulement de l'électricité sont configurés pour s'étendre le long d'une longueur de doigt d'au moins un des un ou plusieurs doigts flexibles au niveau de chacun des un ou plusieurs raccords d'électrodes
dans lequel le circuit électrique flexible, le ou les chemins d'écoulement de l'électricité et les un ou plusieurs doigts flexibles sont de préférence logés au sein d'une partie interne du panneau électrique.
